# EUROPEAN PATENT APPLICATION

(11) **EP 4 177 608 A1**
(43) Date of publication of application: **10.05.2023**
(21) Application number: 21206770.6
(22) Date of filing: 05.11.2021
(51) Int. Cl.: G01N 33/68

(54) **BIOMARKER PANEL FOR DIAGNOSING PULMONARY DYSFUNCTION**

(71) Applicant: Medizinische Hochschule Hannover, 30625 Hannover (DE)
(72) Inventor: Jonigk, Danny, 30625 Hannover (DE); Stark, Helge, 37077 Göttingen (DE); Kühnel, Mark, 30625 Hannover (DE); Länger, Florian, 30559 Hannover (DE)
(74) Representative: Kuttenkeuler, David

(57) **Abstract**

The present invention pertains to a new method for the diagnosis, prognosis, stratification and/or monitoring of a therapy, of a pulmonary dysfunction in a subject. The method is based on the determination of the level of a panel of biomarkers selected from TM4SF18, TRGV9, ADORA2A, H963, IRF2, TNFSF14, TRIB1, SMAD3, TSLP, SLAMF8, THBS1, SOD1, HAS2, TLR2, THBD, TRA, TFEC, SPHK1, COL3A1, Elastin, IL6R, SN, TEK, SOX7, CXCL9, CXCL10, SLC38A6, SLC15A3, TGFA, SLPI, VWF, TLR8, TGFB1, CXCR6, CCL24, PD1, PLA1A, TRD, CTLA4, MMP9, CD301, PDL1, PDL2, TGFB1, CXCR6, CCL24, PD1, PLA1A, TRD, CTLA4, MMP9, and CD301. The new biomarker panels of this invention allow diagnosing and even stratifying various pulmonary dysfunctions in a subject, and even allow early detection of Chronic lung allograft dysfunction (CLAD). Furthermore provided are diagnostic kits for performing the methods of the invention.

## Description

### FIELD OF THE INVENTION

The present invention pertains to a new method for the diagnosis, prognosis, stratification and/or monitoring of a therapy, of a pulmonary dysfunction in a subject. The method is based on the determination of the level of a panel of biomarkers selected from TM4SF18, TRGV9, ADORA2A, H963, IRF2, TNFSF14, TRIB1, SMAD3, TSLP, SLAMF8, THBS1, SOD1, HAS2, TLR2, THBD, TRA, TFEC, SPHK1, COL3A1, Elastin, IL6R, SN, TEK, SOX7, CXCL9, CXCL10, SLC38A6, SLC15A3, TGFA, SLPI, VWT, TLR8, TGFB1, CXCR6, CCL24, PD1, PLA1A, TRD, CTLA4, MMP9, CD301, PDL1, PDL2, TGFB1, CXCR6, CCL24, PD1, PLA1A, TRD, CTLA4, MMP9, and CD301. The new biomarker panels of this invention allow diagnosing and even stratifying various pulmonary dysfunctions in a subject, and even allow early detection of Chronic lung allograft dysfunction (CLAD). Furthermore provided are diagnostic kits for performing the methods of the invention.

### DESCRIPTION

Transplantation of a graft organ or tissue from a donor to a host patient is a common medical procedure. To reduce chances of graft rejection, a tissue allograft is usually accompanied by immunosuppressive therapy. However, despite the use of immunosuppressive therapies, organ transplant rejection can occur. Acute graft rejection of allograft tissue is an immune response that involves T-cell recognition of alloantigen in the allograft, co-stimulatory signals, elaboration of effector molecules by activated T cells, and an inflammatory response within the graft. Activation and recruitment of circulating leukocytes to the allograft is a central feature of this process.

Each year, approximately 300-350 lung transplantations are performed in Germany alone, and more than 4000 worldwide. Chronic lung allograft dysfunction (CLAD) occurs in approximately 50% of patients within 5 years after transplantation and is primarily responsible for the poor long-term outcome of lung transplantation (LTx) compared with other solid organ grafts. CLAD represents an umbrella term for the manifestations of allo-/auto-immune, mechanical, ischaemic and infectious injuries to the graft and encompasses several clinically and morphologically distinct subforms. CLAD is clinically defined by a sustained decline of respiratory function test results. This criterion is rather unspecific, though, because alternative causes of functional impairment, such as acute cellular rejection (ACR) or infection, have to be excluded by simultaneous histological and microbiological testing. Moreover, lung function testing as a diagnostic parameter is relatively insensitive, because a significant amount of functional lung capacity has to be (irrevocably) lost before becoming clinically apparent. CLAD usually manifests 2 to 3 years after LTx, presenting as a variable, but relentless, deterioration of lung function with poor response to the few therapeutic modalities available.

The most prevalent morphological feature in CLAD patients is a progressive fibrous remodelling of the small airways, termed bronchiolitis obliterans (BO). BO is thought to be triggered by a combination of nonimmune bronchial injury and alloimmune and autoimmune mechanisms. Because of its patchy and discontinuous distribution, BO frequently escapes histological detection by transbronchial biopsies (TBBs), rendering conventional tissue-based diagnostics in CLAD patients insensitive.

The second most prevalent morphological feature in CLAD patients is a collagenous obliteration of alveoli with elastosis summarised as alveolar fibroelastosis (AFE). However, neither the patterns for BO nor for AFE are specific for lung allografts, as respiratory dysfunction with very similar morphological injury patterns has been described following haematopoietic stem cell transplantation (HSCT), radio/chemotherapy (RC) and in idiopathic PPFE (iPPFE).

Because currently available treatment options, like macrolide antibiotics, cytoreductive drugs, or switch of immunosuppressive regimens, usually allow stabilization but not normalization of pulmonary function, emphasis has been placed on early detection, and potential prevention, of CLAD. Therefore, additional biomarkers culled from blood, lavage, or tissue would be valuable as diagnostic adjuncts to conventional histology and lung function testing.

US20210040559A1 discloses methods for diagnosis of lung diseases, such as interstitial lung diseases (ILDs). Compositions and kits useful in carrying out a method for diagnosis of lung diseases are also provided.

US20170227549A1 provides biomarkers for rejection of organ transplants, including lung transplants.

WO 2011/112749 discloses a semi-invasive method for characterizing lung injury by analyzing the expression of various chemokines, such as CXCL10 and CXCL9.

WO 2018/046615 and WO 2019/002247 disclose methods and compositions for predicting chronic lung allograft dysfunction based on analysis of POU2AF1 (POU Class 2 Associating Factor), BLK (B-cell lymphocyte kinase) or TCL1A (T-cell leukemia/lymphoma protein 1A) in blood samples of patients having received a lung transplantation.

Jonigk et al. (2015) disclose that the expression level of Interleukin-6 receptor subunit alpha (IL6R), matrix metalloproteinase 1 (MMPi), SMA and Mad-related protein 1 (SMADi), thrombospondin 1 (THBSi), and bone morphogenetic protein 4 (BMP4) in lung biopsy specimens is useful for early diagnosis of chronic lung allograft dysfunction (CLAD) (Jonigk et al. (2015) Molecular Profiling in Lung Biopsies of Human Pulmonary Allografts to Predict Chronic Lung. Am J Pathol. 2015 Dec; 185(12): 3178-88 Allograft Dysfunction).

Due to the continuing need for quick, but sensitive and specific biomarkers suitable for the development of effective and improved diagnostic, prognostic and therapeutic modalities, the present invention seeks to provide a novel approach for a simple but specific and sensitive test system for the diagnosis or monitoring of various pulmonary dysfunctions in a subject. It is therefore an aim of the present invention to provide novel biomarkers and biomarker panels for use as diagnostic and/or prognostic markers and/or for use in the development of novel therapeutics for pulmonary dysfunction, such as chronic lung allograft dysfunction (CLAD). There is also a continuing need for alternative and sensitive biomarkers for early diagnosis of CLAD before symptoms become clinically apparent to allow treatment modalities prior to irrevocable loss of functional lung capacity.

### BRIEF DESCRIPTION OF THE INVENTION

Generally, and by way of brief description, the main aspects of the present invention can be described as follows:

In **a first aspect,** the invention pertains to a method for the diagnosis, prognosis, stratification and/or monitoring of a therapy, of a pulmonary dysfunction in a subject.

In **a second aspect,** the invention pertains to a method for assessing the risk of developing a pulmonary dysfunction in a subject.

In **a third aspect,** the invention pertains to a method for evaluating the treatment success of a patient who received a therapy for treating pulmonary dysfunction.

In **a fourth aspect,** the invention pertains to a diagnostic kit for performing a method according to the present invention.

### DETAILED DESCRIPTION OF THE INVENTION

In the following, the elements of the invention will be described. These elements are listed with specific embodiments, however, it should be understood that they may be combined in any manner and in any number to create additional embodiments. The variously described examples and preferred embodiments should not be construed to limit the present invention to only the explicitly described embodiments. This description should be understood to support and encompass embodiments which combine two or more of the explicitly described embodiments or which combine the one or more of the explicitly described embodiments with any number of the disclosed and/or preferred elements. Furthermore, any permutations and combinations of all described elements in this application should be considered disclosed by the description of the present application unless the context indicates otherwise.

In the first aspect, the invention pertains to a method for the diagnosis, prognosis, stratification and/or monitoring of a therapy, of a pulmonary dysfunction in a subject, comprising the steps of:
(a) Providing a biological sample from the subject, and
(b) Determining the level of at least one biomarker selected from the group consisting of Transmembrane 4 L6 family member 18 (TM4SF18), T cell receptor gamma variable 9 (TRGV9), Adenosine receptor A2a (ADORA2A), Probable G-protein coupled receptor 171 (H963), Interferon regulatory factor 2 (IRF2), Tumor necrosis factor ligand superfamily member 14 (TNFSF14), Tribbles homolog 1 (TRIBi), Mothers against decapentaplegic homolog 3 (SMAD3), Thymic stromal lymphopoietin (TSLP), SLAM family member 8 (SLAMF8), Thrombospondin-i (THBSi), Superoxide dismutase 1 (SODi), Hyaluronan synthase 2 (HAS2), Toll-like receptor 2 (TLR2), Thrombomodulin (THBD), Transformer-2 protein homolog alpha (TRA), Transcription factor EC (TFEC), Sphingosine kinase 1 (SPHKi), Collagen alpha-i(III) chain (COL3A1), Elastin (ELN), Interleukin-6 receptor subunit alpha (IL6R), Sialoadhesin (SN), Angiopoietin-i receptor (TEK), Transcription factor SOX-7 (SOX7), C-X-C motif chemokine 9 (CXCL9), C-X-C motif chemokine 10 (CXCL10), Probable sodium-coupled neutral amino acid transporter 6 (SLC38A6), Solute carrier family 15 member 3 (SLC15A3), Protransforming growth factor alpha (TGFA), Antileukoproteinase (SLPI), von Willebrand factor (VWF), Toll-like receptor 8 (TLR8), Transforming Growth Factor Beta 1 (TGFB1), C-X-C Motif Chemokine Receptor 6 (CXCR6), C-C Motif Chemokine 24 (CCL24), Programmed cell death 1 (PD1), Phospholipase A1 member A (PLA1A), Tetratricopeptide repeat-containing protein (TRD), Cytotoxic T-lymphocyte protein 4 (CTLA4), Matrix metalloproteinase-9 (MMP9), CD301, Programmed cell death 1 ligand 1 (PDL1), Programmed cell death 1 ligand 2 (PDL2), in the biological sample,
wherein a differential level of the at least one biomarker as determined in step (b) compared to a healthy control or reference value is indicative for the presence of the pulmonary dysfunction in the subject.

A "diagnosis" or the term "diagnostic" in context of the present invention means identifying the presence or nature of a pathologic condition. Diagnostic methods differ in their sensitivity and specificity. The "sensitivity" of a diagnostic assay is the percentage of diseased individuals who test positive (percent of "true positives"). Diseased individuals not detected by the assay are "false negatives." Subjects who are not diseased and who test negative in the assay, are termed "true negatives." The "specificity" of a diagnostic assay is 1 minus the false positive rate, where the "false positive" rate is defined as the proportion of those without the disease who test positive. While a particular diagnostic method may not provide a definitive diagnosis of a condition, it suffices if the method provides a positive indication that aids in diagnosis.

The term "prognosis" refers to a forecast as to the probable outcome of the disease as well as the prospect of recovery from the disease as indicated by the nature and symptoms of the case. Accordingly, a negative or poor prognosis is defined by a lower post-treatment survival term or survival rate. Conversely, a positive or good prognosis is defined by an elevated post-treatment survival term or survival rate. Usually prognosis is provided as the time of progression free survival or overall survival.

The term "stratification" for the purposes of this invention refers to the advantage that the method according to the invention renders possible decisions for the treatment and therapy of the patient, whether it is the hospitalization of the patient, the use, effect and/or dosage of one or more drugs, a therapeutic measure or the monitoring of a course of the disease and the course of therapy or etiology or classification of a disease, e.g., into a new or existing subtype or the differentiation of diseases and the patients thereof.

The term "monitoring a therapy" means for the purpose of the present invention to observe disease progression in a subject who receives a therapy. In other words, the subject during the therapy is regularly monitored for the effect of the applied therapy, which allows the medical practitioner to estimate at an early stage during the therapy whether the prescribed treatment is effective or not, and therefore to adjust the treatment regime accordingly.

As used herein, the term "subject" or "patient" refers to any animal (e.g., a mammal), including, but not limited to, humans, non-human primates, rodents, and the like, which is to be the recipient of a particular treatment. Typically, the terms "subject" and "patient" are used interchangeably herein in reference to a human subject. In a particularly preferred embodiment, the subject is a mammal, such as a mouse, a rat, a guinea pig, a rabbit, a cat, a dog, a monkey, or a human, preferably a human, such as a human patient, more preferably a human patient at risk of suffering from a pulmonary dysfunction, and most preferably a human patient having received a lung transplantation, a lung allograft, a graft of hematopoetic stem cells, a chemotherapy, and/or a radiotherapy, optionally wherein said human patient is at risk of suffering from a pulmonary dysfunction.

As used herein, the term "subject at risk of suffering from a pulmonary dysfunction" or "human patient at risk of suffering from a pulmonary dysfunction" refers to a subject that presents one or more symptoms indicative of a pulmonary dysfunction. In one embodiment, the term "subject at risk of suffering from a pulmonary dysfunction" shall refer to a patient having received a lung transplantation and being at risk of suffering from a post-lung transplantation complication, such as organ rejection, acute organ rejection, or organ injury, such as CLAD. In another embodiment, the term "subject at risk of suffering from a pulmonary dysfunction" shall refer to a patient having received a stem cell transplantation and being at risk from suffering from a post-hematopoietic stem cell transplantation complication. Yet another embodiment relates to a patient having received a chemotherapy and/or a radiotherapy and being at risk of suffering from a pulmonary dysfunction associated with chemotherapy and/or radiotherapy.

The term "biological sample" as used herein refers to a sample that was obtained and may be assayed for any one of the biomarkers as disclosed with the present invention, or their gene expression. The biological sample can include a biological fluid (e.g., blood, cerebrospinal fluid, urine, plasma, serum), tissue biopsy, and the like.

A "biomarker" or "marker" in the context of the present invention refers to an organic biomolecule, particularly a polypeptide, a gene, and/or mRNA, which is differentially present in a sample taken from subjects having a certain condition as compared to a comparable sample taken from subjects who do not have said condition. For example, a marker can be a polypeptide or polysaccharide (having a particular apparent molecular weight) which is present at an elevated or decreased level in samples of patients suffering from a pulmonary dysfunction compared to samples of patients with a negative diagnosis.

The term "determining the level of' a biomarker in a sample, control or reference, as described herein shall refer to the quantification of the presence of said biomarkers in the tested sample. For example, the concentration of the biomarkers in said samples may be directly quantified via measuring the amount of a protein/polypeptide/polysaccharide as present in the tested sample. However, also possible is to quantify the amount of biomarker indirectly via assessing the gene expression of the encoding gene of the biomarker, for example by quantification of the expressed mRNA encoding for the respective biomarker. The present invention shall not be restricted to any particular method for determining the level of a given biomarker, but shall encompass all means that allow for a quantification, or estimation, of the level of said biomarker, either directly or indirectly. "Level" in the context of the present invention is therefore a parameter describing the absolute amount of a biomarker in a given sample, for example as absolute weight, volume, or molar amounts; or alternatively "level" pertains to the relative amounts, for example and preferably the concentration of said biomarker in the tested sample, for example mol/l, g/l, g/mol etc. In preferred embodiments the "level" refers to the concentration of the tested biomarkers in g/l.

In some aspects and embodiments of the present invention it may be preferred that the level of said at least one biomarker in said sample is determined by means of a nucleic acid detection method or a protein detection method. However, nucleic acid detection methods are only applicable where an expressed protein is the biomarker. Generally, all means shall be comprised by the present invention which allow for a quantification of the expression of any one of the herein disclosed biomarker. Therefore, also promoter analysis and procedures assessing the epigenetic status of a gene locus encoding a protein biomarker of the invention are comprised by the herein described invention.

Detection methods that are preferred in context of the herein described invention the level of said at least one biomarker in said sample is determined by means of a detection method selected from the group consisting of mass spectrometry, mass spectrometry immunoassay (MSIA), antibody-based protein chips, 2-dimensional gel electrophoresis, stable isotope standard capture with anti-peptide antibodies (SISCAPA), high-performance liquid chromatography (HPLC), western blot, cytometry bead array (CBA), protein immuno-precipitation, radio immunoassay, ligand binding assay, and enzyme-linked immunosorbent assay (ELISA), preferably wherein said protein detection method is ELISA. Suitable alternative detection methods for quantification of a biomarker of the invention are known to the skilled artisan.

"Increase" of the level of a biomarker in a sample compared to a control shall in preferred embodiments refer to statistically significant increase in preferred aspects of the invention.

In alternative embodiments of the invention, certain biomarkers as disclosed herein may also be significantly decreased in the event of a pulmonary dysfunction disease in a subject.

In course of the present invention levels of 226 genes of interest and 2 housekeepers in human lung biopsies from the Surveillance Biopsy Study of 43 human patients having received a lung transplantation that were taken at routine intervals after lung transplantation were analyzed. The gene expression profiles were used to train an algorithm to discriminate between either those patients that have Bronchiolitis Obliterans (C1) vs. those that do not ("not-C1"), or between healthy controls (KO), patients with acute rejection (A2), patients with alveolar fibroelastosis (AFE), patients that will develop BO (preCi), those that have BO (C1), as well as endstage Bronchiolitis Obliterans syndrome (BOS). Using a support vector machine (SVM) with radial kernel function model, the inventors identified multiple genes which allow discriminating between those patients that have BO ("C1") vs. those that do not have BO ("not-C1"). Panels comprising these markers can predict patients that will develop CLAD 12 months before symptoms or morphological hallmarks of CLAD arise with 96% precision.

Preferred embodiments of the present invention pertain to panels of a plurality of biomarkers as identified herein for the diagnostic purposes as described. The advantage of combining the biomarkers disclosed herein is an increased sensitivity and/or specificity of the disclosed assays. Hence a preferred embodiment of the invention pertains to the herein disclosed method wherein the at least one biomarker is at least two, three, four, five, six, seven, eight, nine, or ten biomarkers, and/or a combination of at least two, three, four, five, six, seven, eight, nine, or ten biomarkers. Most preferred is that at least 3 biomarkers are used. More preferred is that at least four biomarkers are used. More preferred is that at least 5 biomarkers are used. More preferred is that at least 6 biomarkers are used. More preferred is that at least 7 biomarkers are used. More preferred is that at least 8 biomarkers are used. More preferred is that at least 9 biomarkers are used.

In one embodiment of the herein disclosed invention the at least one biomarker is a combination of at least the three biomarkers TNFSF14, TRIB1, and SMAD3. In addition to determining the level of at least the three biomarkers TNFSF14, TRIB1, and SMAD3, one of the following biomarkers may be, if desired, added to the panel for testing: TM4SF18, TRGV9, ADORA2A, H963, IRF2, TSLP, SLAMF8, THBS1, SOD1, HAS2, TLR2, THBD, TRA, TFEC, SPHK1, COL3A1, Elastin, IL6R, SN, TEK, SOX7, CXCL9, CXCL10, SLC38A6, SLC15A3, TGFA, SLPI, VWF, TLR8, TGFB1, CXCR6, CCL24, PD1, PLA1A, TRD, CTLA4, MMP9, CD301, PDL1, or PDL2.

One specifically preferred panel for use in context of the herein disclosed invention comprises a combination of at least three biomarkers. In this particularly preferred embodiment, the at least one biomarker is a combination of at least the three biomarkers TNFSF14, TRIB1, and SMAD3.

Further preferred is a combination of at least four biomarkers. In this particularly preferred embodiment, the at least one biomarker is a combination of at least four biomarkers, preferably
(i) TNFSF14, TRIB1, SMAD3, and THBS1,
(ii) TNFSF14, TRIB1, SMAD3, and SOD1,
(iii) TNFSF14, TRIB1, SMAD3, and SLAMF8,
(iv) TNFSF14, TRIB1, SMAD3, and IL6R,
(v) TNFSF14, TRIB1, SMAD3, THBS1, and SOD1,
(vi) TNFSF14, TRIB1, SMAD3, SLAMF8, and SOD1,
(vii) TNFSF14, TRIB1, SMAD3, ADORA2A, and TRIB1,
(viii) TNFSF14, TRIB1, SMAD3, SLAMF8, and THBS1, or
(ix) TNFSF14, TRIB1, SMAD3, SLAMF8, THBS1, and SOD1.

In another specifically preferred panel for use in context of the herein disclosed invention, the at least one biomarker is a combination of at least the biomarkers TM4SF18 and TRGV9.

A preferred embodiment of the herein disclosed invention relates to the at least one biomarker, which is a combination of at least four biomarkers, preferably
(i) TM4SF18, TRGV9, ADORA2A and H963,
(ii) TM4SF18, TRGV9, ADORA2A and VWF, or
(iii) TM4SF18, TRGV9, ADORA2A, H963 and VWF.

Using the support vector machine (SVM) with radial kernel function model, the inventors identified multiple biomarker panels comprising at least the 2 markers TM4F18 and TRGV9, in addition to two more biomarkers. These panels allow an accuracy of at least 83% 6 months and 70% 12 months prior clinical symptoms of BO. Addition of further biomarkers to the panels achieved diagnosis with an accuracy of 88% 6 months and 85% 12 months before symptoms or morphological hallmarks of CLAD arise.

In another specifically preferred embodiment of the herein disclosed invention, the at least one biomarker is a combination of at least four biomarkers selected from TNFSF14, HAS2, TSLP, TLR2, THBD, COL3A1, Elastin, IL6R, SN, and TEK.

In yet another preferred embodiment of the herein disclosed invention, the at least one biomarker is a combination of at least TNFSF14, HAS2, TSLP, TLR2, THBD, COL3A1, Elastin, IL6R, SN, and TEK.

In regard to the present invention it may be preferred in some embodiments that the analysis of the marker panel in step (b) of the diagnostic method of the invention is characterized in that the tested marker panel has an apparent area under the curve (AUC) at 95% confidence interval (CI) of at least 60%, preferably at least 65% or more preferably at least 70%. How to determine the AUC is known to the skilled artisan. Alternatively or additionally, the panel of the invention may be characterized by a sensitivity of at least 75%, preferably at least 80%, and a specificity of at least 40%, preferably at least 50%, more preferably at least 60%.

The biomarkers of the invention are in some embodiments preferably protein biomarkers.

In other embodiments, the biomarkers are genetic biomarkers. In one embodiment, the determining the level of the at least one biomarker in step (b) involves determining the level of a gene and/or the mRNA expression of the at least one biomarker in the biological sample.

A further preferred embodiment relates to the herein disclosed method, wherein the biological sample is a sample of a subject comprising a tissue sample or a body liquid sample, such as a Bronchoalveolar lavage (BAL) sample, a nasal swab sample, a blood sample, a serum sample, a plasma sample, a urine sample, a lymph fluid sample, a pleural fluid sample, and/or a brain liquor sample, preferably wherein the biological sample is a tissue sample or a BAL sample.

In some embodiments, the sample can be a group of cells from a lung, such as a group of cells from a transplanted lung and/or from a lung allograft.

Yet another preferred embodiment relates to the herein disclosed method, wherein the pulmonary dysfunction is Bronchiolitis Obliterans (BO), Bronchiolitis Obliterans syndrome (BOS), chronic lung allograft dysfunction (CLAD), alveolar fibroelastosis (AFE), bronchiolitis obliterans with organizing pneumonitis (BOOP), a chronic pulmonary dysfunction, pneumonia, organizing pneumonia, a fibrotic lung disease, idiopathic pulmonary fibrosis (IPF), an immune-mediated lung disease, such as graft versus host disease or scleroderma, sarcoidosis, hypersensitivity pneumonitis (HP), a post-lung transplantation complication, such as organ rejection, acute organ rejection, or organ injury, a post-hematopoietic stem cell transplantation complication, and/or a pulmonary dysfunction associated with chemotherapy and/or radiotherapy.

The biomarker panels as disclosed herein are particular useful in a screening setting for Bronchiolitis Obliterans (BO), Bronchiolitis Obliterans syndrome (BOS), and/or chronic lung allograft dysfunction (CLAD).

In context of the herein disclosed invention several biomarkers where found to be either differentially upregulated or downregulated in the diagnosis for a pulmonary dysfunction compared to healthy subjects. Hence in context of the herein disclosed invention a differential level of a biomarker selected from TM4SF18, TRGV9, ADORA2A, H963, IRF2, TNFSF14, TRIB1, SMAD3, TSLP, SLAMF8, THBS1, SOD1, HAS2, TLR2, THBD, TRA, TFEC, SPHK1, COL3A1, Elastin, IL6R, SN, TEK, SOX7, CXCL9, CXCL10, SLC38A6, SLC15A3, TGFA, SLPI, VWF, TLR8, TGFB1, CXCR6, CCL24, PD1, PLA1A, TRD, CTLA4, CD301, PDL1, and PDL2, in the biological sample from the subject as determined in step **(b)** compared to a healthy control or reference value is a higher level, which is preferably indicative for a positive diagnosis.

On the other hand, a differential level of a biomarker selected from IL6R, ADORA2A, MMP9 and IRF2 in the biological sample from the subject as determined in step (b) compared to a healthy control or reference value is a lower level, which is preferably indicative for a positive diagnosis.

The skilled artisan will understand that numerous methods may be used to select a threshold or reference value for a particular marker or a plurality of markers. In diagnostic aspects, a threshold value may be obtained by performing the assay method on samples obtained from a population of patients having a certain type of pulmonary dysfunction, such as Bronchiolitis Obliterans (BO), Bronchiolitis Obliterans syndrome (BOS), and/or chronic lung allograft dysfunction (CLAD), and from a second population of subjects that do not have the specific pulmonary dysfunction. For prognostic or treatment monitoring applications, a population of patients, all of which have, for example, BO, may be followed for the time period of interest (e.g., six months following diagnosis or treatment, respectively), and then dividing the population into two groups: a first group of subjects that progress to an endpoint (e.g., recurrence of disease, death); and a second group of subjects that did not progress to the end point. These are used to establish "low risk" and "high risk" population values for the marker(s) measured, respectively. Other suitable endpoints include, but are not limited to, 5-year mortality rates.

Once these groups are established, one or more thresholds may be selected that provide an acceptable ability to predict diagnosis, prognostic risk, treatment success, etc. In practice, Receiver Operating Characteristic curves, or "ROC" curves, are typically calculated by plotting the value of a variable versus its relative frequency in two populations (called arbitrarily "disease" and "normal" or "low risk" and "high risk" for example). For any particular marker, a distribution of marker levels for subjects with and without a disease may overlap. Under such conditions, a test does not absolutely distinguish "disease" and "normal" with 100% accuracy, and the area of overlap indicates where the test cannot distinguish "disease" and "normal." A threshold is selected, above which (or below which, depending on how a marker changes with the disease) the test is considered to be "positive" and below which the test is considered to be "negative." The area under the ROC curve (AUC) is a measure of the probability that the perceived measurement may allow correct identification of a condition.

Additionally, thresholds may be established by obtaining an earlier marker result from the same patient, to which later results may be compared. In some aspects, the individuals act as their own "control group." In markers that increase with disease severity or prognostic risk, an increase over time in the same patient can indicate a worsening of disease or a failure of a treatment regimen, while a decrease over time can indicate remission of disease or success of a treatment regimen.

In some embodiments, multiple thresholds or reference values may be determined. This can be the case in so-called "tertile," "quartile," or "quintile" analyses. In these methods, the "disease" and "normal" groups (or "low risk" and "high risk") groups can be considered together as a single population, and are divided into 3, 4, or 5 (or more) "bins" having equal numbers of individuals. The boundary between two of these "bins" may be considered "thresholds." A risk (of a particular diagnosis or prognosis for example) can be assigned based on which "bin" a test subject falls into.

All numeric values are herein assumed to be modified by the term "about," whether or not explicitly indicated. The term "about" generally refers to a range of numbers that one of skill in the art would consider equivalent to the recited value (i.e., having the same function or result). In many instances, the terms "about" may include numbers that are rounded to the nearest significant figure. In particularly preferred embodiments of the invention the term "about" may refer to a deviation of the respective numeric value of a maximum of 20% of the numerical value, however more preferred is 15%, 10%, 5% and most preferred is 4%, 3%, 2%, and most preferred is 1%.

In a further preferred embodiment, the at least one biomarker is detected using a detection reagent specific for at least one mRNA sequence selected from the group consisting of TM4SF18, TRGV9, ADORA2A, H963, IRF2, TNFSF14, TRIB1, SMAD3, TSLP, SLAMF8, THBS1, SOD1, HAS2, TLR2, THBD, TRA, TFEC, SPHK1, COL3A1, Elastin, IL6R, SN, TEK, SOX7, CXCL9, CXCL10, SLC38A6, SLC15A3, TGFA, SLPI, VWF, TLR8, TGFB1, CXCR6, CCL24, PD1, PLA1A, TRD, CTLA4, MMP9, CD301, PDL1, and PDL2.

In another preferred embodiment, the at least one biomarker is detected using a detection reagent specific for at least one protein selected from the group consisting of TM4SF18, TRGV9, ADORA2A, H963, IRF2, TNFSF14, TRIB1, SMAD3, TSLP, SLAMF8, THBS1, SOD1, HAS2, TLR2, THBD, TRA, TFEC, SPHK1, COL3A1, Elastin, IL6R, SN, TEK, SOX7, CXCL9, CXCL10, SLC38A6, SLC15A3, TGFA, SLPI, VWF, TLR8, TGFB1, CXCR6, CCL24, PD1, PLA1A, TRD, CTLA4, MMP9, CD301, PDL1, and PDL2.

Any and all methods and diagnostic means of this invention can be supplemented by conventional histological analysis of sampled specimens, which would allow an earlier and more specific diagnosis of CLAD, making earlier treatment with potentially better outcome possible.

Yet another aspect of this invention relates to a method for assessing the risk of developing a pulmonary dysfunction in a subject, said method comprising the steps:
(a) Obtaining a biological sample from the subject, and
(b) Determining the level of at least one biomarker selected from the group consisting of TM4SF18, TRGV9, ADORA2A, H963, IRF2, TNFSF14, TRIB1, SMAD3, TSLP, SLAMF8, THBS1, SOD1, HAS2, TLR2, THBD, TRA, TFEC, SPHK1, COL3A1, Elastin, IL6R, SN, TEK, SOX7, CXCL9, CXCL10, SLC38A6, SLC15A3, TGFA, SLPI, VWF, TLR8, TGFB1, CXCR6, CCL24, PD1, PLA1A, TRD, CTLA4, MMP9, CD301, PDL1, and PDL2, in the biological sample,
wherein a differential level of the at least one biomarker as determined in step (b) compared to a healthy control or reference value is indicative for an increased risk of the subject for developing a pulmonary dysfunction.

In one preferred embodiment relating to the method for assessing the risk of developing a pulmonary dysfunction in a subject, the pulmonary dysfunction is a chronic pulmonary dysfunction, Bronchiolitis Obliterans (BO), Bronchiolitis Obliterans syndrome (BOS), chronic lung allograft dysfunction (CLAD), alveolar fibroelastosis (AFE), bronchiolitis obliterans with organizing pneumonitis (BOOP), pneumonia, organizing pneumonia, a fibrotic lung diseases, idiopathic pulmonary fibrosis (IPF), an immune-mediated lung disease, such as graft versus host disease or scleroderma, sarcoidosis, hypersensitivity pneumonitis (HP), a post-lung transplantation complication, such as organ rejection, acute organ rejection, or organ injury, a post-hematopoietic stem cell transplantation complication, and/or a pulmonary dysfunction associated with chemotherapy and/or radiotherapy.

Particularly preferred is that the subject is a human having received a lung transplantation, a lung allograft, a graft of hematopoetic stem cells, a chemotherapy, and/or a radiotherapy.

Another preferred embodiment relates to the method for assessing the risk of developing a pulmonary dysfunction in a subject, wherein said healthy control or reference value corresponds to the level of said at least one biomarker in a provided biological sample obtained from the subject before receiving said lung transplantation, lung allograft, graft of hematopoetic stem cells, chemotherapy, and/or radiotherapy.

A further aspect of this invention pertains to a method for evaluating the treatment success of a patient who received a therapy for treating pulmonary dysfunction, comprising
(a) Providing a biological sample from said patient who received a therapy for treating pulmonary dysfunction,
(b) Determining the level of at least one biomarker selected from the group consisting of TM4SF18, TRGV9, ADORA2A, H963, IRF2, TNFSF14, TRIB1, SMAD3, TSLP, SLAMF8, THBS1, SOD1, HAS2, TLR2, THBD, TRA, TFEC, SPHK1, COL3A1, Elastin, IL6R, SN, TEK, SOX7, CXCL9, CXCL10, SLC38A6, SLC15A3, TGFA, SLPI, VWF, TLR8, TGFB1, CXCR6, CCL24, PD1, PLA1A, TRD, CTLA4, MMP9, CD301, PDL1, and PDL2, in the biological sample, and
(c) Comparing the level of said at least one biomarker as determined in (b) with a reference sample or reference value,
wherein a decrease or an increase of the level of at least one biomarker in the biological sample from said subject compared to said reference sample or reference value is an indication for the patient's response to said treatment.

In a preferred embodiment of the method for evaluating the treatment success of a patient who received a therapy for treating pulmonary dysfunction, the reference sample or reference value corresponds to the level of said at least one biomarker in a provided biological sample obtained from said patient before receiving said treatment.

In a preferred embodiment, the method of the herein disclosed invention is performed in form of a non-invasive method, preferably as an *ex vivo* method or *in vitro* method. Since the herein described diagnostic methods are non-invasive the term "providing a biological" sample shall preferably not be interpreted to include a surgical procedure conducted at the subject.)

In yet another aspect, the invention provides kits for aiding a diagnosis of a pulmonary dysfunction in a subject, wherein the kits can be used to detect the biomarkers of the present invention. For example, the kits can be used to detect any one or any combination of biomarkers described above, which biomarkers are differentially present in samples of a patient having the pulmonary dysfunction compared to a control subject. The kits of the invention have many applications. For example, the kits can be used to differentiate whether a subject has the pulmonary dysfunction, or has a negative diagnosis, thus aiding a diagnosis. In another example, the kits can be used to identify compounds that modulate expression of the biomarkers in *in vitro* lung cells or in *vivo* animal models for a pulmonary dysfunction, such as a pig or a sheep.

Preferably the kit of the invention is a diagnostic kit for performing a method in accordance with the present invention comprising means for quantifying the level of said at least one biomarker. Preferably the kit of the invention comprises means for quantifying a biomarker selected from TM4SF18, TRGV9, ADORA2A, H963, IRF2, TNFSF14, TRIB1, SMAD3, TSLP, SLAMF8, THBS1, SOD1, HAS2, TLR2, THBD, TRA, TFEC, SPHK1, COL3A1, Elastin, IL6R, SN, TEK, SOX7, CXCL9, CXCL10, SLC38A6, SLC15A3, TGFA, SLPI, VWF, TLR8, TGFB1, CXCR6, CCL24, PD1, PLA1A, TRD, CTLA4, MMP9, CD301, PDL1, and PDL2. Such means for quantifying is for example an antibody or antibody-like molecule.

One aspect of this invention relates to a diagnostic kit comprising a detection reagent specific for at least one biomarker selected from the group consisting of TM4SF18, TRGV9, ADORA2A, H963, IRF2, TNFSF14, TRIB1, SMAD3, TSLP, SLAMF8, THBS1, SOD1, HAS2, TLR2, THBD, TRA, TFEC, SPHK1, COL3A1, Elastin, IL6R, SN, TEK, SOX7, CXCL9, CXCL10, SLC38A6, SLC15A3, TGFA, SLPI, VWF, TLR8, TGFB1, CXCR6, CCL24, PD1, PLA1A, TRD, CTLA4, MMP9, CD301, PDL1, and PDL2.

In a preferred embodiment, the diagnostic kit comprises detection reagents specific for a combination of at least two, three, four, five, six, seven, eight, nine, or ten, preferably four or five, mRNA sequences selected from the group consisting of TM4SF18, TRGV9, ADORA2A, H963, IRF2, TNFSF14, TRIB1, SMAD3, TSLP, SLAMF8, THBS1, SOD1, HAS2, TLR2, THBD, TRA, TFEC, SPHK1, COL3A1, Elastin, IL6R, SN, TEK, SOX7, CXCL9, CXCL10, SLC38A6, SLC15A3, TGFA, SLPI, VWF, TLR8, TGFB1, CXCR6, CCL24, PD1, PLA1A, TRD, CTLA4, MMP9, CD301, PDL1, and PDL2. Alternatively, the diagnostic kit comprises detection reagents specific for a combination of at least two, three, four, five, six, seven, eight, nine, or ten, preferably four or five, proteins selected from the group consisting of TM4SF18, TRGV9, ADORA2A, H963, IRF2, TNFSF14, TRIB1, SMAD3, TSLP, SLAMF8, THBS1, SOD1, HAS2, TLR2, THBD, TRA, TFEC, SPHK1, COL3A1, Elastin, IL6R, SN, TEK, SOX7, CXCL9, CXCL10, SLC38A6, SLC15A3, TGFA, SLPI, VWF, TLR8, TGFB1, CXCR6, CCL24, PD1, PLA1A, TRD, CTLA4, MMP9, CD301, PDL1, and PDL2. For example, the kit can comprise antibodies specific for a combination of at least two, three, four, five, six, seven, eight, nine, or ten, preferably four or five, proteins selected from the group consisting of TM4SF18, TRGV9, ADORA2A, H963, IRF2, TNFSF14, TRIB1, SMAD3, TSLP, SLAMF8, THBS1, SOD1, HAS2, TLR2, THBD, TRA, TFEC, SPHK1, COL3A1, Elastin, IL6R, SN, TEK, SOX7, CXCL9, CXCL10, SLC38A6, SLC15A3, TGFA, SLPI, VWF, TLR8, TGFB1, CXCR6, CCL24, PD1, PLA1A, TRD, CTLA4, MMP9, CD301, PDL1, and PDL2.

Optionally, the kit can further comprise instructions for suitable operational parameters in the form of a label or a separate insert. For example, the kit may have standard instructions informing a consumer how to wash the probe after a sample of plasma is contacted on the probe.

In either embodiment, the kit may optionally further comprise a standard or control information so that the test sample can be compared with the control information standard to determine if the test amount of a marker detected in a sample is a diagnostic amount consistent with a diagnosis of a pulmonary dysfunction in the subject.

The terms "of the [present] invention", "in accordance with the invention", "according to the invention" and the like, as used herein are intended to refer to all aspects and embodiments of the invention described and/or claimed herein.

As used herein, the term "comprising" is to be construed as encompassing both "including" and "consisting of', both meanings being specifically intended, and hence individually disclosed embodiments in accordance with the present invention. Where used herein, "and/or" is to be taken as specific disclosure of each of the two specified features or components with or without the other. For example, "A and/or B" is to be taken as specific disclosure of each of (i) A, (ii) B and (iii) A and B, just as if each is set out individually herein. In the context of the present invention, the terms "about" and "approximately" denote an interval of accuracy that the person skilled in the art will understand to still ensure the technical effect of the feature in question. The term typically indicates deviation from the indicated numerical value by ±20%, ±15%, ±10%, and for example ±5%. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect. Where an indefinite or definite article is used when referring to a singular noun, e.g. "a", "an" or "the", this includes a plural of that noun unless something else is specifically stated.

It is to be understood that application of the teachings of the present invention to a specific problem or environment, and the inclusion of variations of the present invention or additional features thereto (such as further aspects and embodiments), will be within the capabilities of one having ordinary skill in the art in light of the teachings contained herein.

Unless context dictates otherwise, the descriptions and definitions of the features set out above are not limited to any particular aspect or embodiment of the invention and apply equally to all aspects and embodiments which are described.

All references, patents, and publications cited herein are hereby incorporated by reference in their entirety.

### BRIEF DESCRIPTION OF THE FIGURES

The figures show:

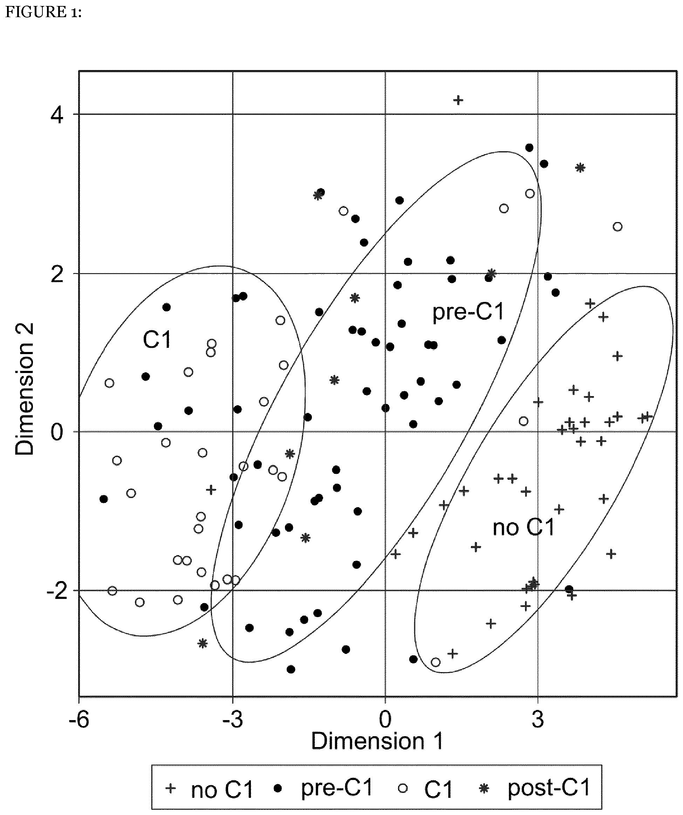

**Figure 1** shows the 2-diminsional visualization (t-SNA) of merged training and validation data of the discrimination analysis of gene profiles in human lung biopsies. Biopsies were analyzed histologically and by molecular biology, and healthy controls (no C1), patients that will develop BO (preCi), patients that have BO (C1), as well as patients that suffered from BO (postC1) are depicted. Of note, patients that have BO (C1) are classified as chronic rejection according to ISHLT classification.

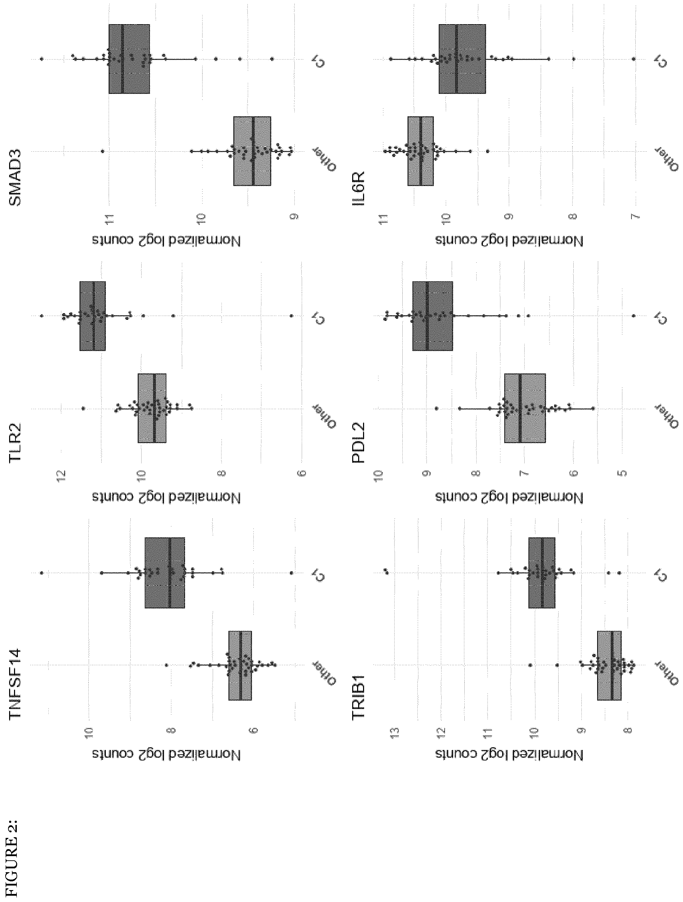

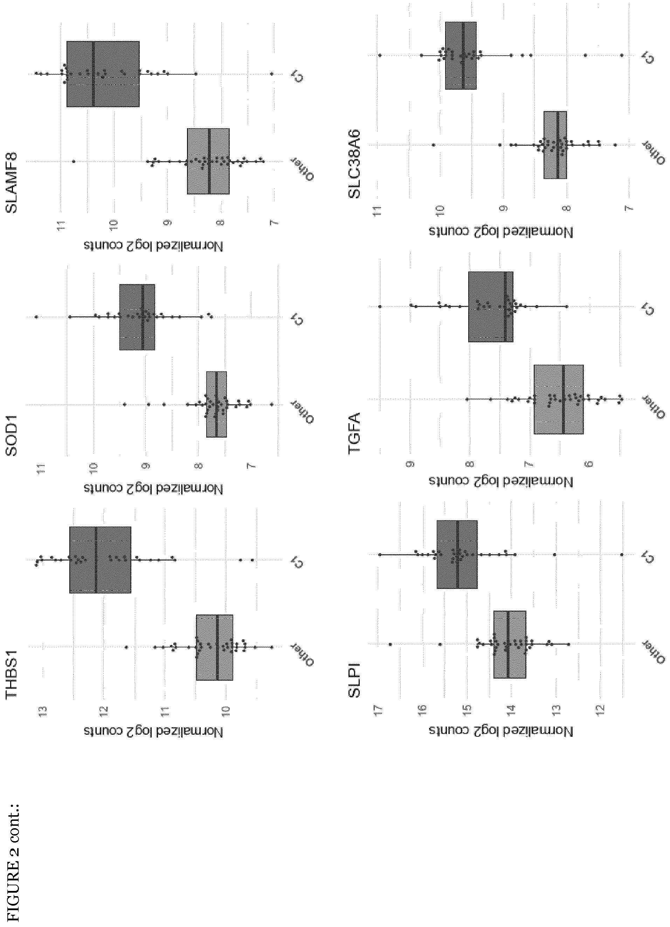

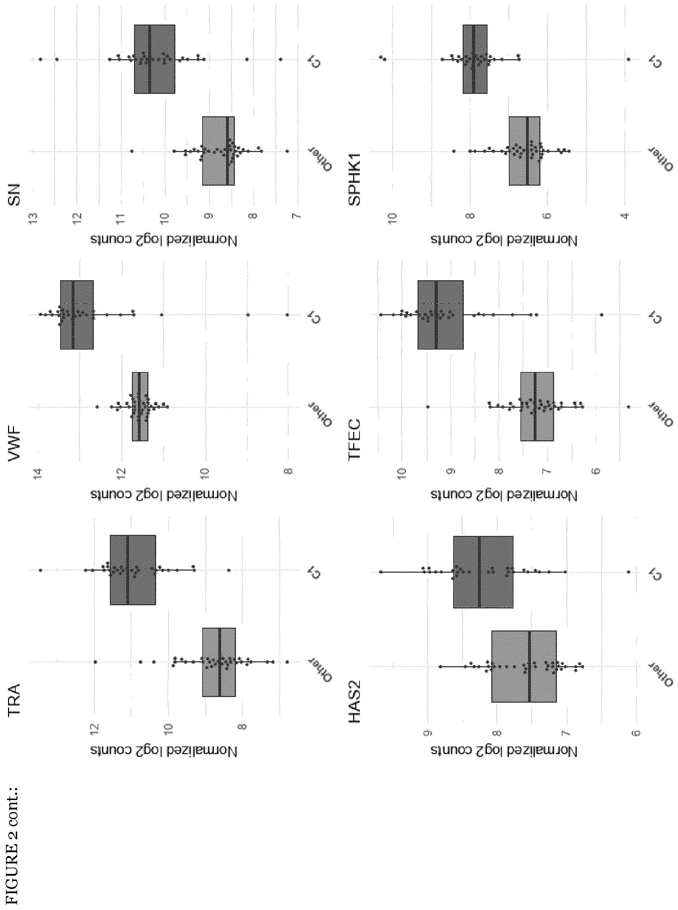

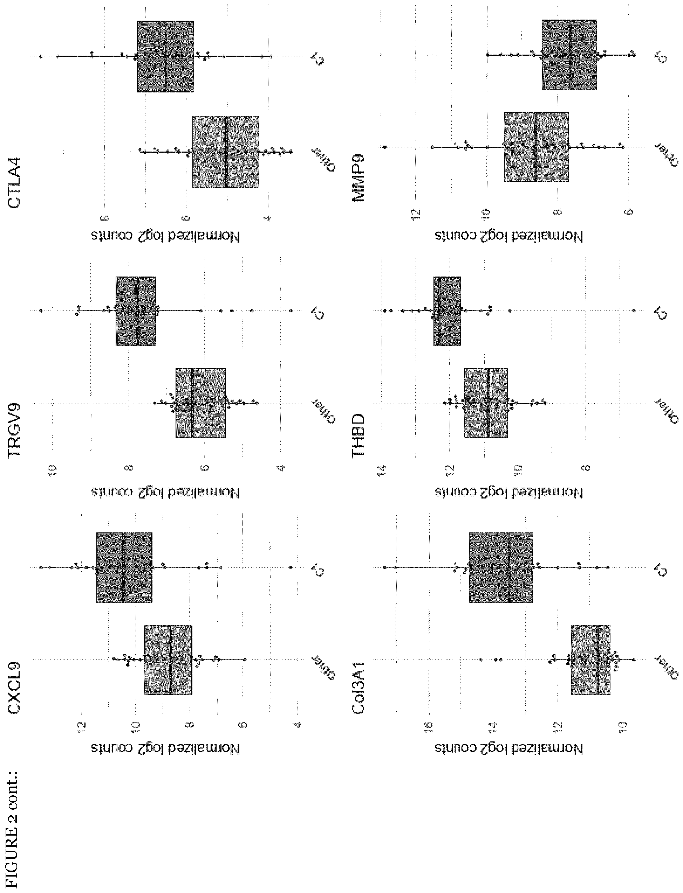

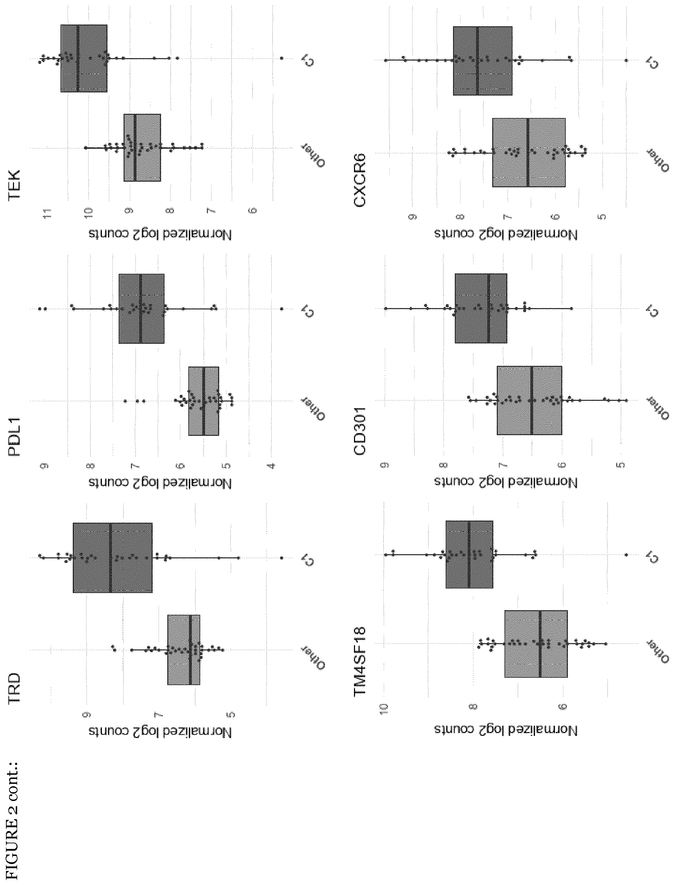

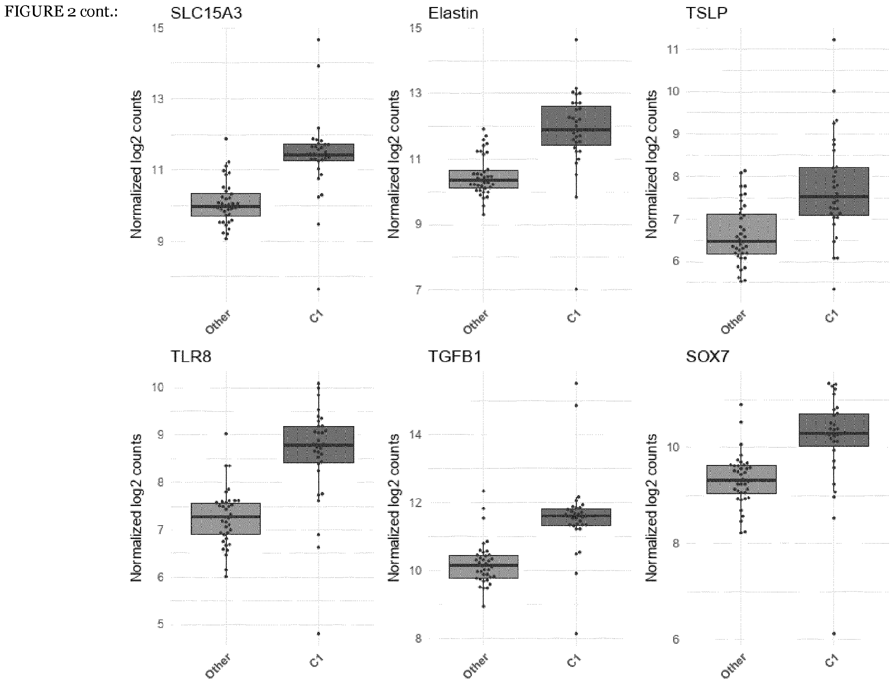

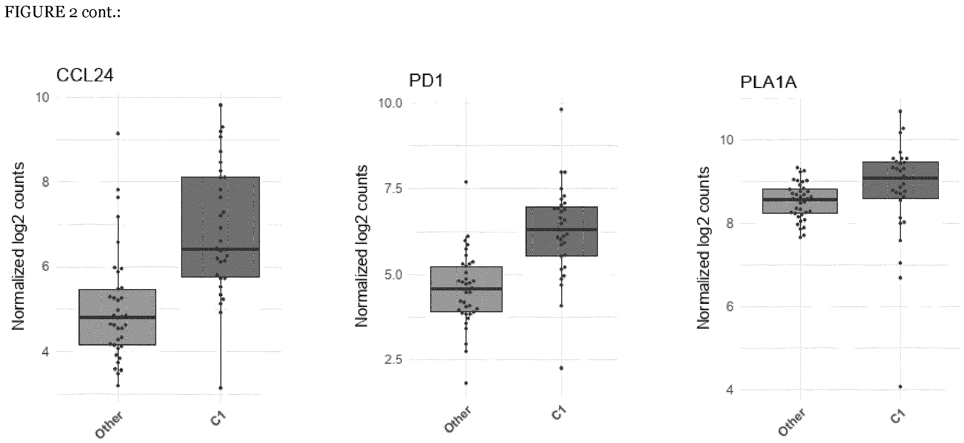

**Figure 2**: shows the expression of genes in a first group that has BO ("C1") and a second group that does not have BO ("other"), analysed by support vector machine with radial kernel function (10 genes were used as input), normalized log2 counts. Classification of genes: Card.pairwise.6. Asterisks (*/**/***/****) represent the significance level between the two groups (Mann-Whitney-U test, fdr corrected).

### EXAMPLES

Certain aspects and embodiments of the invention will now be illustrated by way of example and with reference to the description, figures and tables set out herein. Such examples of the methods, uses and other aspects of the present invention are representative only, and should not be taken to limit the scope of the present invention to only such representative examples.

The examples show:

### Example 1: Gene analysis of Surveillance Biopsy Study samples of human patients having received a lung transplantation

Biopsies from the Surveillance Biopsy Study of human patients having received a lung transplantation were taken at routine intervals after lung transplantation and analyzed using a gene expression panel based on Nanostring technology comprising a custom gene panel including 226 genes of interest and 2 housekeepers (POL2a, GAPDH). The discrimination analysis of gene profiles in human lung biopsies from 43 patients that were analyzed histologically and by molecular biology are shown in Figure 1. In particular, healthy controls (KO), patients with acute rejection (A2), patients with alveolar fibroelastosis (AFE), patients that will develop BO (preC1) and those that have BO (C1), as well as endstage Bronchiolitis Obliterans syndrome (BOS) are depicted. Of note, green samples are often found together with blue ones. These are most likely cases where the biopsy did not show the BO morphologically, but the molecular profiles indicates the presence of BO.

Figure 2 shows the first attempt to discriminate between these groups for training and testing models using the gene expression panel including the 226 genes of interest and the 2 housekeepers. The first attempt achieved only an accuracy of around 40%. Therefore, the inventors reduced the discrimination to a simpler classification, namely discriminating between a first group comprising healthy controls (KO (grey)) and patients with acute rejection (A2 (red)), compared to those that have BO (C1 (green)) vs. those that do not have BO, but did not suffer from an acute organ rejection ("not-C1") (see Figure 3). Figure 3 depicts 37 KO and A2 samples and 31 C1 samples, analyzed by a training/testing model (50/50). This analysis depicts models with high accuracy during training and testing with an accuracy of up to 91% on the training set and 97% on the test set. The model as shown in Figure 3 classifies 100% of BOS as C1, 81% of preC1 as C1, 93% of preC1 as C1 (up to 12 months prior to CLAD, 27 samples), and 75% of postC1 as C1, whereas classification of AFE is ambiguous (50/50).

### Example 2: Development of machine learning algorithms

Various machine learning algorithms were trained to distinguish samples with ISHLT C1 status (31 samples) and CO status, i.e. "not-C1" (37 samples). The resulting models are very accurate both during training and against a large test set (50% of the available data withheld for testing). The models accurately predict a C1 status before a histopathological diagnosis of a C1 status can be made (tested on 57 samples). The accuracy increases if the time interval until a histopathological diagnosis of the C1 status is small (≤ 12 months, tested on 27 samples). The inventors discovered that the most successful algorithm is a support vector machine (SVM) with radial kernel function.

Using the support vector machine (SVM) with radial kernel function model, the inventors identified multiple genes, which allow discriminating between those patients that have BO (C1) vs. those that do not have BO ("not-C1"). These genes are suitable for diagnosing BO, in particular in combination panels comprising at least 3 of those genes. These panels can predict patients that will develop CLAD 12 months before symptoms or morphological hallmarks of CLAD arise with 96% precision. The panels of this invention therefore allow a diagnosis that is 9times less likely to be a false positive or false negative diagnosis of CLAD.

**Table 1 lists biomarkers of the present invention.**

| Protein Name | Gene Name | UniProtKB ID No. |
|---|---|---|
| Transmembrane 4 L6 family member 18 | TM4SF18 | Q96CE8 |
| T cell receptor gamma variable 9 | TRGV9 | Q99603 |
| Adenosine receptor A2a | ADORA2A | Q99604 |
| Probable G-protein coupled receptor 171 | H963 | O14626 |
| Interferon regulatory factor 2 | IRF2 | P14316 |
| Tumor necrosis factor ligand superfamily member 14 | TNFSF14 | O43557 |
| Tribbles homolog 1 | TRIB1 | Q96RU8 |
| Mothers against decapentaplegic homolog 3 | SMAD3 | P84022 |
| Thymic stromal lymphopoietin | TSLP | Q969D9 |
| SLAM family member 8 | SLAMF8 | Q9P0V8 |
| Thrombospondin-1 | THBS1 | P07996 |
| Superoxide dismutase 1 | SOD1 | P00441 |
| Hyaluronan synthase 2 | HAS2 | Q92819 |
| Toll-like receptor 2 | TLR2 | O60603 |
| Thrombomodulin | THBD | P07204 |
| Transformer-2 protein homolog alpha | TRA | Q13595 |
| Transcription factor EC | TFEC | O14948 |
| Sphingosine kinase 1 | SPHK1 | Q9NYA1 |
| Collagen alpha-i(III) chain | COL3A1 | P02401 |
| Elastin | ELN | P15502 |
| Interleukin-6 receptor subunit alpha | IL6R | P08887 |
| Sialoadhesin | SN | Q9BZZ2 |
| Angiopoietin-1 receptor | TEK | Q02763 |
| Transcription factor SOX-7 | SOX7 | Q9BT81 |
| C-X-C motif chemokine 9 | CXCL9 | Q07325 |
| C-X-C motif chemokine 10 | CXCL10 | P02778 |
| Probable sodium-coupled neutral amino acid transporter 6 | SLC38A6 | Q8IZM9 |
| Solute carrier family 15 member 3 | SLC15A3 | Q8IY34 |
| Protransforming growth factor alpha | TGFA | P01135 |
| Antileukoproteinase | SLPI | P03973 |
| von Willebrand factor | VWF | P04275 |
| Toll-like receptor 8 | TLR8 | Q9NR97 |
| Programmed cell death 1 ligand 1 | PDL1 | Q9NZQ7 |
| Programmed cell death 1 ligand 2 | PDL2 | Q9BQ51 |
| Transforming growth factor beta-i proprotein | TGFB1 | P01137 |
| C-X-C chemokine receptor type 6 | CXCR6 | O00574 |
| C-C motif chemokine 24 | CCL24 | O00175 |
| Programmed cell death protein 1 | PD1 | Q15116 |
| Phospholipase A1 member A | PLA1A | Q53H76 |
| T cell receptor delta constant | TRD | B7Z8K6 |
| Cytotoxic T-lymphocyte protein 4 | CTLA4 | P16410 |
| Matrix metalloproteinase-9 | MMP9 | P14780 |
| C-type lectin domain family 10 member A | CD301 | Q8IUN9 |

**Table 2 lists 29 genes which are used for building the models, with the following frequency and averaged levels of importance (ranging from 0 to 100).**

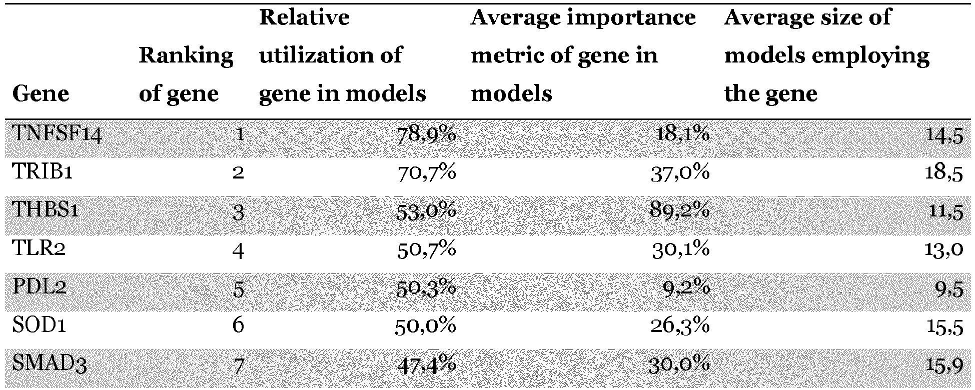

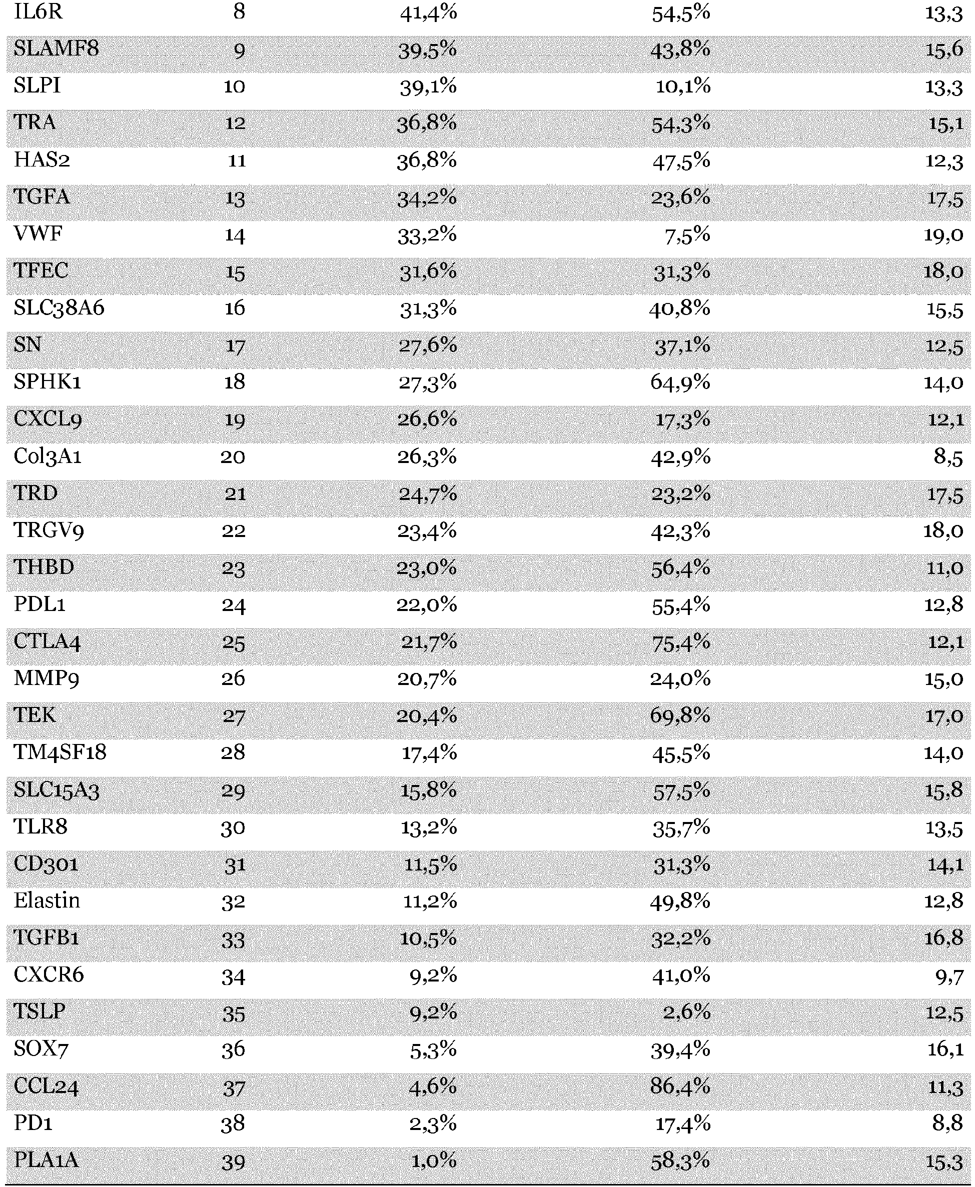

### Example 3: Development of a diagnostic panel of 6 biomarkers

Using a panel comprising the 6 biomarkers THBS1, SOD1, SLAMF8, TRIB1, SMAD3, and TNFSF14, a discrimination between patients that have BO ("C1") and those that do not ("not-C1"/"other") with high accuracy is possible. In particular, the inventors observed that a discrimination between a first group of patients that have BO (C1) and a second group comprising all other indications ("not-C1"/"other"), i.e. healthy controls, patients with acute rejection, patients with alveolar fibroelastosis (AFE), those that will develop BO (preCi), as well as endstage BO syndrome (BOS (yellow)) is possible using the 6 biomarker panel comprising THBS1, SOD1, SLAMF8, TRIB1, SMAD3, and TNFSF14 with an accuracy of ∼95%.

**Table 3: Panel comprising the 6 biomarkers THBS1, SOD1, SLAMF8, TRIB1, SMAD3, and TNFSF14**

| **Algorithm** | Support Vector Machine (SVM) with radial kernel |
|---|---|
| **Genes in model** | 6 |
| **Accuracy (training)** | 94,3% |
| *(samples without rejection vs. samples with chronic rejection, used in training; 50% of overall data used)* | |
| **Accuracy (validation)** | 90,9% |
| *(samples without rejection vs. samples with chronic rejection, never used in training; remaining 50% of overall data used)* | |
| **Accuracy (early detection)** | 94,7% |
| *(all samples before manifestation of chronic rejection, never used in training; 57 samples)* | |
| **Accuracy (early detection)** | 96,3% |
| *(samples up to 52 weeks before manifestation of chronic rejection, never used in training; 27 samples)* | |
| **Accuracy (early detection)** | 94,40% |
| *(samples up to 26 weeks before manifestation of chronic rejection, never used in training; 18 samples)* | |

### References

The references are:
1. Jonigk et al. (2015) Molecular Profiling in Lung Biopsies of Human Pulmonary Allografts to Predict Chronic Lung. Am J Pathol. 2015 Dec; 185(12): 3178-88 Allograft Dysfunction).

## Claims

1. **A method for the diagnosis, prognosis, stratification and/or monitoring of a therapy, of a pulmonary dysfunction in a subject,** comprising the steps of:
**(a)** Providing a biological sample from the subject, and
**(b)** Determining the level of at least one biomarker selected from the group consisting of TM4SF18, TRGV9, ADORA2A, H963, IRF2, TNFSF14, TRIB1, SMAD3, TSLP, SLAMF8, THBS1, SOD1, HAS2, TLR2, THBD, TRA, TFEC, SPHK1, COL3A1, Elastin, IL6R, SN, TEK, SOX7, CXCL9, CXCL10, SLC38A6, SLC15A3, TGFA, SLPI, VWF, TLR8, TGFB1, CXCR6, CCL24, PD1, PLA1A, TRD, CTLA4, MMP9, CD301, PDL1, and PDL2, in the biological sample,
wherein a differential level of the at least one biomarker as determined in step **(b)** compared to a healthy control or reference value is indicative for the presence of the pulmonary dysfunction in the subject.

2. The method according to claim 1, wherein the at least one biomarker is a combination of at least the three biomarkers TNFSF14, TRIB1, and SMAD3.

3. The method according to claim 1 or 2, wherein the at least one biomarker is a combination of at least four biomarkers, preferably
(i) TNFSF14, TRIB1, SMAD3, and THBS1,
(ii) TNFSF14, TRIB1, SMAD3, and SOD1,
(iii) TNFSF14, TRIB1, SMAD3, and SLAMF8,
(iv) TNFSF14, TRIB1, SMAD3, and IL6R,
(v) TNFSF14, TRIB1, SMAD3, THBS1, and SOD1,
(vi) TNFSF14, TRIB1, SMAD3, SLAMF8, and SOD1,
(vii) TNFSF14, TRIB1, SMAD3, ADORA2A, and TRIB1,
(viii) TNFSF14, TRIB1, SMAD3, SLAMF8, and THBS1, or
(ix) TNFSF14, TRIB1, SMAD3, SLAMF8, THBS1, and SOD1.

4. The method according to claim 1, wherein the at least one biomarker is a combination of at least the biomarkers TM4SF18 and TRGV9.

5. The method according to claim 1 or 4, wherein the at least one biomarker is a combination of at least four biomarkers, preferably
(i) TM4SF18, TRGV9, ADORA2A and H963,
(ii) TM4SF18, TRGV9, ADORA2A and VWF, or
(iii) TM4SF18, TRGV9, ADORA2A, H963 and VWF.

6. The method according to claim 1, wherein the at least one biomarker is a combination of at least four biomarkers selected from TNFSF14, HAS2, TSLP, TLR2, THBD, COL3A1, Elastin, IL6R, SN, and TEK.

7. The method according to claim 1 or 6, wherein the at least one biomarker is a combination of at least TNFSF14, HAS2, TSLP, TLR2, THBD, COL3A1, Elastin, IL6R, SN, and TEK.

8. The method according to any one of claims 1 to 7, wherein the biological sample is a sample of a subject comprising a tissue sample or a body liquid sample, such as a Bronchoalveolar lavage (BAL) sample, a nasal swab sample, a blood sample, a serum sample, a plasma sample, a urine sample, a lymph fluid sample, a pleural fluid sample, and/or a brain liquor sample, preferably wherein the biological sample is a tissue sample or a BAL sample.

9. The method according to any one of claims 1 to 8, wherein the pulmonary dysfunction is Bronchiolitis Obliterans (BO), Bronchiolitis Obliterans syndrome (BOS), chronic lung allograft dysfunction (CLAD), alveolar fibroelastosis (AFE), bronchiolitis obliterans with organizing pneumonitis (BOOP), a chronic pulmonary dysfunction, pneumonia, organizing pneumonia, a fibrotic lung disease, idiopathic pulmonary fibrosis (IPF), an immune-mediated lung disease, such as graft versus host disease or scleroderma, sarcoidosis, hypersensitivity pneumonitis (HP), a post-lung transplantation complication, such as organ rejection, acute organ rejection, or organ injury, a post-hematopoietic stem cell transplantation complication, and/or a pulmonary dysfunction associated with chemotherapy and/or radiotherapy.

10. The method according to any one of claims 1 to 9, wherein a differential level of a biomarker selected from TM4SF18, TRGV9, ADORA2A, H963, IRF2, TNFSF14, TRIB1, SMAD3, TSLP, SLAMF8, THBS1, SOD1, HAS2, TLR2, THBD, TRA, TFEC, SPHK1, COL3A1, Elastin, IL6R, SN, TEK, SOX7, CXCL9, CXCL10, SLC38A6, SLC15A3, TGFA, SLPI, VWF, TLR8, TGFB1, CXCR6, CCL24, PD1, PLA1A, TRD, CTLA4, CD301, PDL1, and PDL2, in the biological sample from the subject as determined in step **(b)** compared to a healthy control or reference value is a higher level, and/or wherein a differential level of the biomarker IL6R, ADORA2A, MMP9 or IRF2 in the biological sample from the subject as determined in step **(b)** compared to a healthy control or reference value is a lower level.

11. **A method for assessing the risk of developing a pulmonary dysfunction in a subject, said method comprising the steps:**
**(a)** Obtaining a biological sample from the subject, and
**(b)** Determining the level of at least one biomarker selected from the group consisting of TM4SF18, TRGV9, ADORA2A, H963, IRF2, TNFSF14, TRIB1, SMAD3, TSLP, SLAMF8, THBS1, SOD1, HAS2, TLR2, THBD, TRA, TFEC, SPHK1, COL3A1, Elastin, IL6R, SN, TEK, SOX7, CXCL9, CXCL10, SLC38A6, SLC15A3, TGFA, SLPI, VWF, TLR8, TGFB1, CXCR6, CCL24, PD1, PLA1A, TRD, CTLA4, MMP9, CD301, PDL1, and PDL2, in the biological sample,
wherein a differential level of the at least one biomarker as determined in step **(b)** compared to a healthy control or reference value is indicative for an increased risk of the subject for developing a pulmonary dysfunction.

12. The method according to claim 11, wherein the subject is a human having received a lung transplantation, a lung allograft, a graft of hematopoetic stem cells, a chemotherapy, and/or a radiotherapy, optionally wherein said healthy control or reference value corresponds to the level of said at least one biomarker in a provided biological sample obtained from the subject before receiving said lung transplantation, lung allograft, graft of hematopoetic stem cells, chemotherapy, and/or radiotherapy.

13. **A method for evaluating the treatment success of a patient who received a therapy for treating pulmonary dysfunction,** comprising
**(a)** Providing a biological sample from said patient who received a therapy for treating pulmonary dysfunction,
**(b)** Determining the level of at least one biomarker selected from the group consisting of TM4SF18, TRGV9, ADORA2A, H963, IRF2, TNFSF14, TRIB1, SMAD3, TSLP, SLAMF8, THBS1, SOD1, HAS2, TLR2, THBD, TRA, TFEC, SPHK1, COL3A1, Elastin, IL6R, SN, TEK, SOX7, CXCL9, CXCL10, SLC38A6, SLC15A3, TGFA, SLPI, VWF, TLR8, TGFB1, CXCR6, CCL24, PD1, PLA1A, TRD, CTLA4, MMP9, CD301, PDL1, and PDL2, in the biological sample, and
**(c)** Comparing the level of said at least one biomarker as determined in **(b)** with a reference sample or reference value,
wherein a decrease or an increase of the level of at least one biomarker in the biological sample from said subject compared to said reference sample or reference value is an indication for the patient's response to said treatment.

14. The method according to claim 13, wherein said reference sample or reference value corresponds to the level of said at least one biomarker in a provided biological sample obtained from said patient before receiving said treatment.

15. **A diagnostic kit** for performing a method according to any of the preceding claims, the diagnostic kit comprising a detection reagent specific for at least one mRNA sequence selected from the group consisting of TM4SF18, TRGV9, ADORA2A, H963, IRF2, TNFSF14, TRIB1, SMAD3, TSLP, SLAMF8, THBS1, SOD1, HAS2, TLR2, THBD, TRA, TFEC, SPHK1, COL3A1, Elastin, IL6R, SN, TEK, SOX7, CXCL9, CXCL10, SLC38A6, SLC15A3, TGFA, SLPI, VWF, TLR8, TGFB1, CXCR6, CCL24, PD1, PLA1A, TRD, CTLA4, MMP9, CD301, PDL1, and PDL2.
